# EUROPEAN PATENT SPECIFICATION

(11) **EP 4 393 457 B1**
(45) Date of publication and mention of the grant of the patent: **04.02.2026**
(21) Application number: 22383299.9
(22) Date of filing: 28.12.2022
(51) Int. Cl.: A61F 13/00, A61F 13/02

(54) **WOUND DRESSING FOR DETECTING AN INFECTION IN WOUND EXUDATE**
WUNDVERBAND ZUR ERKENNUNG EINER INFEKTION IN WUNDEXSUDAT
PANSEMENT POUR LA DÉTECTION D'UNE INFECTION PAR UN EXSUDAT DE PLAIE

(43) Date of publication of application: 03.07.2024
(73) Proprietor: Paul Hartmann AG, 89522 Heidenheim (DE)
(72) Inventor: Gonzalez Gómez-Arevalillo, Fernando, 08030 Basconia 69, Barcelona (ES); Zúñiga Ruiz, Camilo Javier, 08025 Barcelona (ES); Yagüe, Jose Luis, 08025 Barcelona (ES); Ben-Aissa Soler, Alejandra, 08302 Mataró, Barcelona (ES); Moya Lara, Ana, 08302 Mataró, Barcelona (ES); Delgado Simao, Claudia Custodia, 08014 Barcelona (ES); Pozuelo Ruiz, Marta, 43008 Tarragona (ES); del Bas, Josep Maria, 43007 Tarragona (ES)
(74) Representative: Paul Hartmann AG Patents & Licensing

(56) References cited:
- CA-A1- 2 333 107
- US-A1- 2014 018 654
- US-A1- 2021 361 490
- US-A1- 2022 370 000

## Description

The invention relates to a wound dressing comprising a support layer adapted for enzymatic detection of an infection in a wound exudate.

A wound can be regarded as separation of the contiguity of tissues of the skin, wherein this can be combined with a loss of substance.

The healing of wounds is based on the ability of the skin to regenerate tissue such as epithelial tissue, connective and supporting tissue. The regeneration of tissue is a complex occurrence of cell activities overlapping each other, wherein said cell activities promote the healing process step by step. An accumulation of wound exudate, which might contain inter alia blood, proteins, residues of cells, microorganisms and leucocytes, can promote the growth of bacteria. Such bacteria can be for example Staphylococcus aureus and Pseudomonas aeruginosa being present on the skin or widespread in moist milieu. These bacteria cause infection and thus delay the healing of the wound.

Keeping this in mind and in order to improve the wound healing by appropriate handling or treatment, the state of the wound with regard to the grade of infections should be detected, the earlier the better.

Wound fluid - often also designated as exudate or wound exudate - is typically a liquid that is secreted by the wound and whose composition generally reflect the inflammation and colonization status of the wound. Wound fluid may amongst others also contain cells of the patient and bacteria.

For on-site testing of an analyte suspected to be present in a medical sample of a patient, it is important to minimise the number of steps, the number of test components and the amount of reagent handling. Many commercially available medical tests consist of a sampler and a transportation unit to transport the newly taken sample to the laboratory for closer analyses. However, this practice has many drawbacks since it puts demands on the sampler, transportation medium and the transportation unit itself. Moreover, it generally leads to an inevitable delay in receiving an assay result from the laboratory.

In order to overcome these deficiencies different kinds of on-site tests have been developed. There are some known, self-contained assay devices which carry a reagent (test substance) which detects the analyte by reacting therewith. A positive result may be indicated, for example, by a visible change of a colour. In general, this type of assay device carries the reagent on a matrix material to which the sample is subsequently added for testing.

Well-known examples of this type of assay device are pregnancy tests and tests to determine protein, proteolytic enzymes and leukocytes in urinary samples.

Wound dressings with integrated test functions are also known. By using such wound dressings, it is possible to determine the status of a wound infection directly at the wound site without removing the dressing. Such wound dressings are known, for example, from WO 2014/011207 Al.

However, it is often observed that the test substance, which is applied to the carrier matrix, for example a gauze, diffuses laterally and thus no sharp delimitation of the reaction field is possible. This problem is even more apparent in test systems where several reagent substances are applied next to each other and a colour change in response to the analyte is observed. The diffusion can lead to the test liquids mixing with each other and thus to errors in evaluation.

In addition, there is a desire to record and evaluate the test strips digitally, for example with a smartphone. Such a digital recording and evaluation of the colour changes caused by the test reaction requires precise application and fixation of test substances in clearly defined areas of the carrier so that overlaps are avoided.

EP 3 435 941 B1 discloses a wound dressing with at least one test substance which is able to show a colour change, whereby the test substance is bound to a matrix via a molecular anchor. This method requires that the test substance must be chemically modified, which is time-consuming and cost-intensive.

It is therefore the object of the present invention to overcome the disadvantages inherent in the prior art and to provide a wound dressing for detecting an infection on-site that is easy to manufacture and allows a precise detection of the colour change occurring after the test reaction.

The problem is solved by a wound dressing comprising a support layer adapted for detecting an infection in a wound exudate. The support layer comprises a carrier material that is capable of conducting liquids. The carrier material has a first surface and a second surface opposite the first surface. The carrier material comprises at least one test area to which a test substance is applied, wherein the carrier material further comprises a diffusion barrier. The diffusion barrier prevents lateral diffusion of the test substance, such that the test substance is retained within the at least one test area by the diffusion barrier.

By the invention it is achieved that the test substance does not have to be chemically modified. Instead, the test substance can be applied to the carrier material in a liquid form without further modification. The liquid can only diffuse up to the diffusion barrier and cannot spread beyond it in the carrier material. As a result, such a support layer, in contrast to the support layers known from the state of the art, is compatible with a variety of test substances without any further chemical modifications.

The support layer according to the invention is easy and inexpensive to produce. Moreover, it allows a sharp delineation of different types of test substances that can be applied to the same sample of carrier material. Thus, a wound dressing according to the invention can be provided in a cost-efficient manner.

According to the invention, a support layer is understood to be any layer which is suitable to carry a system for detecting infections in wound exudate. Alternatively, it can also be referred to as a "carrier layer".

Preferably, the support layer may be a sheet-like layer, wherein the first surface and the second surface have a substantially parallel course.

Also the carrier material, which is capable of conducting liquids, may be a sheet-like layer, wherein the two surfaces have a substantially parallel course.

The term "substantially" is to be interpreted in such a way that irregularities may occur, for example due to the manufacture of materials, so that the parallel course of the two surfaces, i.e. the first surface and the second surface, deviates from parallelism only in limited areas.

When the support layer is in an intended state of use, namely integrated into a wound dressing, which is applied to a wound, the first surface typically faces away from the wound and the second surface faces towards the wound. According to the invention, the surface on which the test substance is applied is considered to be the first surface.

By means of the diffusion barrier, the at least one test area is defined. According to the invention, a test area is understood to be a region or portion on or in the carrier material which is delimited from other regions on or in the carrier material by the diffusion barrier. The at least one test area may either be completely surrounded by a diffusion barrier or separated from another portion of the carrier material by the diffusion barrier in such a way that a liquid cannot pass to the other side of the diffusion barrier and thus from one portion to another.

In the context of the present invention, "diffusion" is understood to mean any penetration or spreading of usually liquid substances into the carrier material. This can take place, for example, by compensating for differences in concentration, in particular by capillary effects, but is not limited to these physical effects.

Wound exudate - often also designated as exudate or wound fluid - is typically a liquid that is secreted by the wound and whose composition generally reflects the inflammation and colonization status of the wound. Wound fluid may amongst others also contain cells of the patient, blood and bacteria.

A parameter, also referred to as biological parameter, biomarker or biological marker is a measurable indicator of some biological state or condition. Biomarkers are often measured and evaluated using blood, urine, or soft tissues to examine normal biological processes, pathogenic processes, or pharmacologic responses to a therapeutic intervention. Examples of biological parameters are the pH value or any kind of blood values. A particularly preferred biological parameter to be measured in the context of the present invention in wound exudate is a parameter related to the status of a wound. The measurement can be based on enzymes present in wound exudate which are associated with an inflammatory response of the human body due to a wound infection. Accordingly, such a measurement may indicate the infection status of a wound.

The support layer comprises a carrier material or completely consists of a carrier material. The carrier material is capable of conducting liquids. This means that the liquids can penetrate the carrier material and disperse in the carrier material, for example by capillary forces.

Therefore, the carrier material comprises or consists of an absorbent material, a paper, a hydrogel, a non-woven textile, a woven textile or a foam. It may also comprise or consist of a combination of said materials. It is particularly preferred that the carrier material comprises or consists of a gauze. The support layer may be configured as a wound pad.

It has been shown that the carrier material according to the invention may be based on the same kind of materials that are typically used for wound dressings, such as for example materials comprising cellulose, cellulose derivatives, or absorbent synthetic or natural fibres. This allows the support layer to be easily integrated into wound dressings.

Preferably, the diffusion barrier has hydrophobic properties. It is essential to the invention that the material of which the diffusion barrier is made prevents liquids from overcoming or breaking through the diffusion barrier.

The diffusion barrier comprises a hydrophobic substance, a polymeric material and/or a lipid. Preferably, the diffusion barrier may comprise one or more silicones, in particular polydimethylsiloxane.

Preferably, the diffusion barrier is printed, plotted, stamped, sprayed or coated onto the carrier material, in particular onto the first surface of the carrier material. Preferably the diffusion barrier is screen printed onto the first surface of the carrier material.

According to a preferred embodiment, the diffusion barrier is generated by applying a printable liquid to the carrier material in a manufacturing process. The printable liquid comprises a liquid barrier composition. After application of the liquid barrier composition to the carrier material the liquid dries or cures, thus forming the diffusion barrier. This allows the diffusion barrier to be generated using printers or other liquid applicators present in production lines. As a result, the support layer according to the invention can be produced simply and inexpensively. The barrier composition may contain solvents and dries after application to the carrier material. Alternatively, the barrier composition may comprise a monomer or an uncured polymer that cures after application to the carrier material.

Since the barrier composition is in liquid form for manufacturing, it penetrates the carrier material after application and then dries or cures. The longer the time to cure or dry, the deeper the diffusion barrier can extend into the carrier material. Preferably, the diffusion barrier extends substantially completely through the carrier material in a vertical direction from the first surface to the second surface. In general, the diffusion barrier only needs to extend into the material far enough to prevent the test substance from spreading out of the test area. Thus, the depth of penetration of the diffusion barrier into the substrate may be dependent on the amount of test substance applied.

The diffusion barrier is tubular and act in the manner of a channel. The diffusion barrier can direct wound fluid from the second surface of the carrier material to the first surface of the carrier material.

Therefore, according to the invention, the diffusion barrier extends tubular from the first surface towards the second surface of the carrier material.

**In** an embodiment, the diffusion barrier may extend at least 10 %, preferably at least 50 %, and particularly preferably substantially completely into the carrier material from the first surface to the second surface in a vertical direction.

Viewed from the side to which the diffusion barrier has been applied, i.e. measured at the first surface of the carrier material, the thickness of the wall of the diffusion barrier may be between 0.1 mm and 5.0 mm, preferably between 0.5 mm and 3.0 mm and more preferably between 0.6 mm and 2.0 mm.

In the case where the at least one test area is circular, the diameter of the test area may be 1 mm to 20 mm, preferably 2 mm to 10 mm and more preferably 3 mm to 5 mm, also measured at the first surface of the carrier material. This enables a good evaluation of a reaction of the test substance in the test area, for example with the naked eye, with a photo detector or with any other suitable sensor device.

Preferably the carrier material may have a thickness between 0.5 mm and 10.0 mm, more preferably between 1.0 mm and 8.0 mm and in particular between 1.5 mm and 5.0 mm. This allows the test substance to be integrated into materials that are commonly used for wound dressings. The thickness or layer thickness of the carrier material is defined as the distance from the first surface to the second surface of the carrier material.

The carrier material may comprise 10 % to 100 % viscose. Preferably, it consists of 100 % viscose. However, a material made of 100 % cotton or mixed fabrics of cotton and viscose can also be used as carrier material. In the case of blended fabrics, the cotton content can preferably be between 10 % and 90 %.

In general, synthetic and natural materials or mixtures of synthetic and natural materials are suitable as carrier material. However, materials that can absorb liquids are preferred.

In a preferred embodiment the carrier material may comprise 85 % viscose fibres and 15 % PE/PET fibres.

To determine parameters of wound, in particular to detect wound infections, the test substance may comprise a compound which is selected from the group consisting of Fast Blue RR, N-(Methoxysuccinyl)-Ala-Ala-Pro-Val-p-nitroanilide, N-Succinyl-Ala-Ala-Ala-p-nitroanilide or PG-RBB, preferably TMB and particularly preferably Guaiacol, and any combination thereof.

It is preferred that the test substance is suitable to react with a target enzyme. The target enzyme may be selected from the group consisting of lysozyme, cathepsin G, elastase, catalase, lipase, and esterase, in particular myeloperoxidase (MPO), and any combination thereof. The selection of such a test substance allows to determine the status of a wound and thus an infection with the support layer according to the invention. As the support layer is integrated into a wound dressing, the determination of the wound status is possible on-site, i.e. without the wound dressing having to be removed from the wound. This leads to rapid results and there is thus the possibility of taking immediate and appropriate therapeutic measures. This can reduce the hospitalisation rate and thus also the costs for the health care system.

In a particularly preferred embodiment, the carrier material comprises at least 2, preferably 2 to 50, more preferably 2 to 10 test areas.

Preferably, the test areas are round. This enables the arrangement of a large number of test areas on the carrier material. A punctual application of the test substance can also be easily implemented by machine and is therefore preferred. Preferably, the test substance is applied to the carrier material by drop casting.

In principle, the invention is not limited to the number of test areas arranged on the support layer. Preferably, at least four test areas are applied to the carrier material. Thus, at least two test areas can be provided with one test substance and at least two further test areas with another test substance. This increases the probability that the body fluid to be tested reaches a test area and reacts with the test substance.

Between 5 % and 90 %, preferably between 10 % and 85 % and more preferably between 20 % and 80 % of the first surface may be covered by the at least one test area. This means that the support layer can have one large test area, but also several test areas of different or the same size. The size of the test area is determined by the course of the diffusion barrier.

In a further embodiment, each of the test areas comprises only one type of test substance. However, it is particularly preferred that different types of test substances are applied several times, i.e. that, for example, two test areas are provided with a first type of test substance and two further test areas are provided with a second type of test substance, whereby the first and second type of test substances are different types of test substances, i.e. they differ from each other in their composition and/or function.

For example, each of the test areas may comprise only one type of test substance or the test areas may comprise at least two different types of test substances.

In a particularly preferred embodiment the test areas comprise at least two different types of test substances. This increases the precision and reliability of the detection of wound infections, since one or the other test substance might act as a back-up in case that one of the detection systems fails or has very low sensitivity and specificity. On the other hand, having one test substance in several test areas increase the likelihood that the exudate comes into contact with the test substance and therefore there would be a quick response to a wound infection.

It is preferred that the test substance is capable of producing a detectable signal, in particular a visible colour change upon interaction with the target enzyme. This makes visual reading possible by a user of the wound dressing. For example, a person can visually detect a colour change on the support layer without having to remove the wound dressing. Using a suitable test substance or a suitable combination of test substances in the test areas, a characterization of the body fluid underneath the wound dressing can thus be made easily and quickly.

The visible colour change may also be recorded and evaluated digitally, for example with a smartphone.

To indicate in which area the at least one test area is located, the at least one test area can be surrounded by a visually visible marking line.

The wound dressing can be, for example, a wound plaster, a bandage, a cover of a negative pressure wound treatment bandage used in negative pressure wound therapy ("npwt") or a first-aid dressing.

The wound dressing can further comprise a wound contacting layer and/or a backing layer and/or an absorbent layer. According to the invention the support layer is in fluid communication with the wound contacting layer and the backing layer overlays the first surface of the support layer.

Such a wound dressing offers the advantage that biological parameters and therefore an infection of the wound can be determined in the wound exudate without removing the wound dressing. Thus, there is a focused transfer of information from the second surface of the support layer to the first surface of the support layer.

The invention also relates to a method for manufacturing a support layer for a wound dressing according to the invention.

In a first step a carrier material, which is capable of conducting liquids and has a first surface and a second surface opposite the first surface, is provided. Next, a barrier composition comprising a hydrophobic substance is applied onto on the first surface of the carrier material. The barrier composition is capable of forming a diffusion barrier in the carrier material so that a test area confined by said diffusion barrier is formed. The barrier composition is cured and/or dried. A test substance is applied to the test area on the first surface, such that the test substance is retained within the test area by the diffusion barrier. Thus, the diffusion barrier prevents lateral spreading of the test substance and a precise confinement of the test substance is achieved.

The method according to the invention makes it possible to easily produce a support layer for test substances without having to modify the test substances. As a result, the support layer according to the invention is versatile and can be provided with all common test substances since the diffusion barrier prevents the test substances from running or spreading in the carrier material.

The diffusion barrier allows the test area to be designed in any desired shape since the test area may be determined by the application pattern of the diffusion barrier.

Preferably, the diffusion barrier is applied to the substrate in a circular shape so that the circular area, i.e. the area surrounded by the diffusion barrier, corresponds to the test area.

In an alternative embodiment, the diffusion barrier is applied in such a way that the test area represents a symbol or logo. This allows an individual design and marking of the support layer, for example by applying the diffusion barrier in such a way that the test area takes the form of a company logo. Likewise, a design of the test areas as a lettering is conceivable. In addition, the shapes of the test areas can be combined with each other as desired.

To build the diffusion barrier the barrier composition may be inkjet printed, plotted, sprayed or coated by slot die, in particular screen printed, onto the carrier material. Applying the barrier composition by means of a printing process enables accelerated and cost-effective manufacture. In addition, known materials can be used as carrier materials, which also simplifies manufacture and reduces costs.

The printing process also allows the use of conventional printers from this field without having to change the printers beyond the usual settings.

For the same reason also the test substance may be applied to the test area by a printing process, for example inkjet printing, plotting, spraying or coating by slot die. Preferably, the test substance is applied to the test area by drop casting.

The invention and further advantageous embodiments thereof are described and explained in more detail below with reference to the examples shown in the drawings. The features to be taken from the description and the drawings can be applied individually or in any combination in accordance with the invention. The figures show:
FIG. 1 a schematic top view of a first embodiment of a support layer with diffusion barriers surrounded by a marking line,
FIG. 2 a schematic SEM image of the first surface of the support layer with the diffusion barrier,
FIG. 3 a schematic sectional view along the line A-A of the support layer according to Fig. 1,
FIG. 4 a schematic SEM image of the sectional view according to FIG. 3, and
FIG. 5 a schematic sectional view of a support layer integrated in a wound dressing.

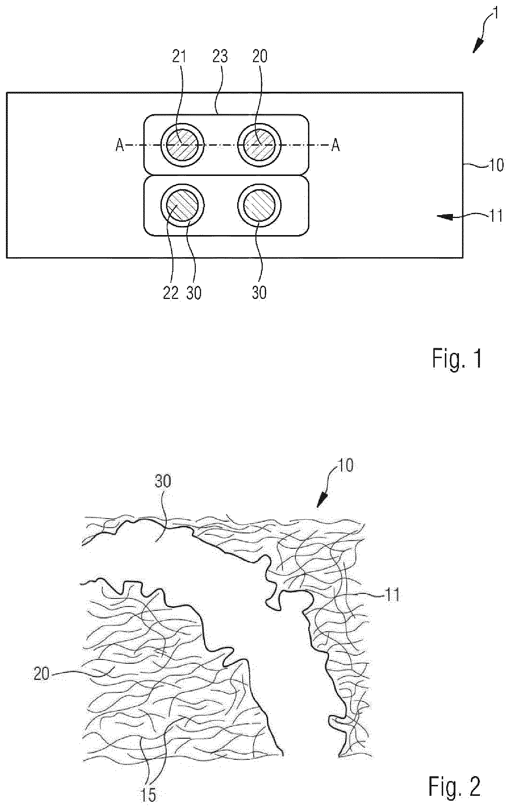

Figure 1 shows a schematic top view of the first surface 11 of a carrier material 10 of a support layer 1. The support layer 1 comprises the carrier material 10, which in particular is a gauze. In the example shown the carrier material 10 has four diffusion barriers 30. The barriers are made from a material containing a silicone. The diffusion barriers 30 each enclose a test area 20 in a circular shape. Each two test areas 20 or diffusion barriers 30 are surrounded by a visually recognisable coloured marking line 23. This creates visually separated test fields. The marking line 23 serves to indicate to a user of the support layer 1 where the test areas 20 are located. However, the diffusion barrier 30 may also comprise a dye and thus the marking line 23 may be omitted. The test substances 21, 22 may also be optically visible, so that a marking line 23 need not be provided either.

In an alternative embodiment (not shown) each diffusion barrier 30 is individually surrounded by the visually recognisable coloured marking line 23.

The user is understood to be any individual who uses the wound dressing 40. The user may be a patient, a nurse or a medical doctor, for example.

In Figure 1, by way of example, the test areas 20 in the upper test field are provided with a test substance 21 and the test areas 20 in the lower test field are provided with a test substance 22. The test substance 21 comprises a pH indicator which changes colour depending on the pH value. The test substance 22 is an indicator substrate which can be converted into a coloured product by an enzyme. Particularly preferably, the test substance 22 in the embodiment according to Figure 1 comprises Guiacol. Guiacol is convertible into a coloured product by the enzyme myeloperoxidase (MPO) in the presence of hydrogen peroxide.

The support layer 1 integrated in the wound dressing 40 is suitable for the detection of infections of a wound.

In the schematical representation of an SEM image shown in figure 2, the diffusion barrier 30 was printed on the first surface 11 of the carrier material 10. Shown in figure 2 is only a quarter of the diffusion barrier 30 surrounding the test area 20. In this embodiment, the carrier material 10 is a non-woven. The diffusion barrier 30 has penetrated the carrier material 10 between and along the fibres 15 by capillary forces. Due to the irregularity caused by the arrangement of the fibres 15, the diffusion barrier 30 is not sharply defined, but has an irregular course. The test area 20 is surrounded by the diffusion barrier 30.

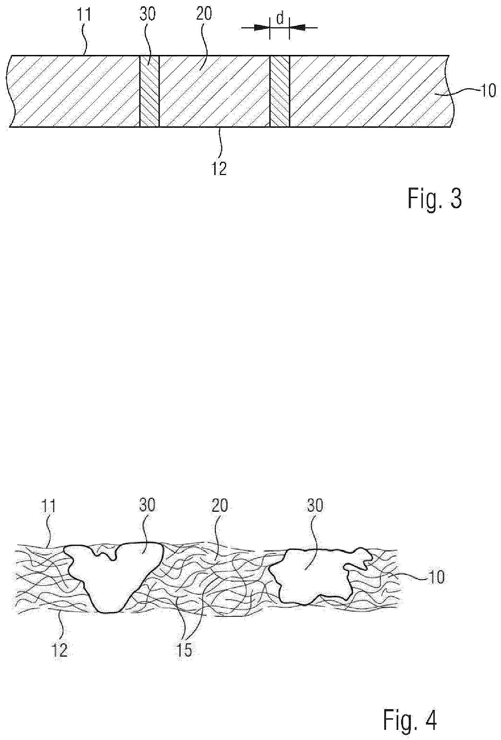

Figure 3 shows a schematic section through the carrier material 10 of Fig. 1 along the line A-A. In this embodiment, the diffusion barrier 30, which surrounds the test area 20 in a circle, extends completely through the carrier material 10 from the first surface 11 to the second surface 12. The diffusion barrier 30 thus extends through the carrier material 10 in a tubular, channel-like or cylindrical manner. Thus, the test area 20 defined by the diffusion barrier 30 also has a three-dimensional extension and provides sufficient lumen for the test substance 21, 22. It can be seen that the test area 20 has a three-dimensional extension along the surfaces 11 and 12 and through the carrier material 10.

The test area 20 itself consists of the carrier material 10, which has absorbent properties. The test area 20 is delimited by the diffusion barrier 30 and by the surfaces 11 and 12.

The thickness d of the diffusion barrier 30 is essentially determined by the manufacturing process of the support layer 1. If the diffusion barrier 30 is applied to the first surface 11 for example by means of a printing process, the thickness d changes in the direction of the second surface 12, since the initially liquid composition from which the diffusion barrier 30 is formed diffuses into the carrier material 10 before it hardens.

The representation of the diffusion barrier 30 in the figures is idealised and schematic. The thickness d changes as the composition penetrates, is absorbed or diffuses into the material until it is sufficiently dry.

Figure 4 is a section through the support layer 1 along the line A-A of Figure 1. Figure 4 is based on a SEM image and shows a real course of the diffusion barrier 30, namely that the diffusion barrier 30 has an irregular course from the first surface 11 to the second surface 12 of the carrier material 10. In this example, the diffusion barrier 30 extends completely along the entire thickness of the carrier material 10. The diffusion barrier 30 has penetrated the carrier material 10 between and along the fibres 15 by capillary forces. Due to the irregularity caused by the arrangement of the fibres 15, the diffusion barrier 30 is not sharply defined, but has an irregular course. The test substance 21, 22 (not shown) can be applied to the test area 20.

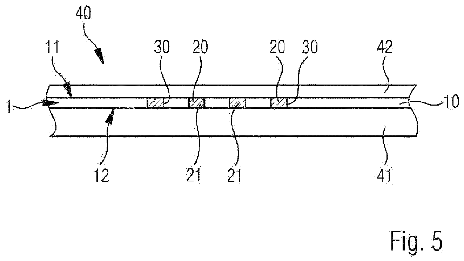

Figure 5 shows the support layer 1 integrated into the wound dressing 40. The support layer 1 is adhered to a backing layer 42 by means of an adhesive (not shown). The backing layer 42 overlays the first surface 11 of the support layer 1 and seals the wound dressing 40. The backing layer 42 is suitably transparent, although it might be slightly opaque, to allow the user to see the test areas 20. The support layer 1 comprises the test areas 20, in each of which a test substance 21 is applied. Optionally, a wound contact layer 41 is arranged on the side of the backing layer 1 facing a wound in the state of use, namely the second surface 12. In this embodiment, the wound contact layer 41 is formed as an absorbent wound pad and is in fluid communication with the support layer 1, so that wound exudate can be absorbed from the wound and passes through the wound contact layer 41 to the support layer 1.

### Example of a support layer and manufacturing of the support layer for the wound dressing

In the following, the manufacturing process of a particularly preferred embodiment of the support layer for a wound dressing according to the invention is described in a non-limiting example.

In a first step, a gauze comprising 85 % viscose fibres and 15 % PE/PET fibres was provided. The barrier composition is prepared by using a two-part liquid silicone kit (Sylgard 184, provided by DOW CORNING), which comprises 12 % w/w of Dimethylvinylated and Trimethylated Silica. The two components are mixed in a 10:1 ratio. The composition cures slowly at room temperature (a complete cure is obtained after 48 hours at 25°C) and more quickly at elevated temperatures (a complete cure is obtained after 35 min at 100°C). The curable polydimethylsiloxane elastomer was deposited on the gauze by screen printing at room temperature (25°C). The stencil for screen printing contains the design of the diffusion barrier, namely circles with an inner diameter of 3 mm in this example. After applying, the barrier composition was cured at 80 °C for 30 min in order to obtain the diffusion barriers, which surrounds the test areas completely. The amount of barrier composition applied was 3,11 mg/cm². It was found that curing the composition at 80 °C for at least 30 min provides a sufficient curing for immediate further processing of the samples, i.e. the application of the test substance.

Additionally, a visually recognisable coloured marking line was screen-printed on the same surface of the gauze as the barrier composition to create visually separated test fields. The marking line serves to indicate to a user of the wound dressing where the test areas are located. To print the marking line a printable dielectric ink (CFSN6057:SUNTRONIC 681 DIELECTRIC:BG04 provided by SunChemical) was used.

3 µL of the test substance was then applied to each of the test areas by drop-casting. An example of the used test substance for detection of infection level of myeloperoxidase (MPO) comprises 24,8 mM guaiacol, 7,35 mM H₂O₂, 45,6 mM PBS pH=7,2 and 200 µM 4-aminobenzoic hydrazide.

## Claims

1. A wound dressing (40) comprising a support layer (1) adapted for enzymatic detection of an infection in a wound exudate, wherein the support layer (1) comprises a carrier material (10) that is capable of conducting liquids, the carrier material (10) comprises or consists of an absorbent material, a paper, a hydrogel, a non-woven textile, a woven textile, a foam, or a gauze, the carrier material (10) has a first surface (11) and a second surface (12) opposite the first surface (11), and wherein the carrier material (10) comprises at least one test area (20) to which a test substance (21, 22) is applied, the carrier material (10) comprises a diffusion barrier (30) which comprises a hydrophobic substance, a polymeric material and/or a lipide and prevents lateral diffusion of the test substance (21, 22), such that the test substance (21, 22) is retained in the at least one test area (20) by the diffusion barrier (30), **characterized in that** the diffusion barrier (30) extends tubularly from the first surface (11) towards the second surface (12) of the carrier material (10).

2. The wound dressing (40) of claim 1, **characterized in that** the diffusion barrier (30) comprises one or more silicones, in particular polydimethylsiloxane.

3. The wound dressing (40) of claim 1 or claim 2, **characterized in that** the diffusion barrier (30) is printed, plotted, stamped, sprayed or coated onto the first surface (11) of the carrier material (10), preferably the diffusion barrier (30) is screen printed onto the first surface (11) of the carrier material (10).

4. The wound dressing (40) of any one of the preceding claims, **characterized in that** the diffusion barrier (30) completely surrounds the at least one test area (20).

5. The wound dressing (40) of any one of the preceding claims, **characterized in that** the diffusion barrier (30) extends at least 10 %, preferably at least 50 %, and particularly preferably substantially completely into the carrier material (10) from the first surface (11) to the second surface (12) in a vertical direction.

6. The wound dressing (40) of any one of the preceding claims, **characterized in that** the thickness (d) of the wall of the diffusion barrier (30) measured at the first surface (11) is between 0.1 mm and 5.0 mm, preferably between 0.5 mm and 3.0 mm and more preferably between 0.6 mm and 2.0 mm.

7. The wound dressing (40) of any one of the preceding claims, **characterized in that** the support layer (1) is configured as a wound pad.

8. The wound dressing (40) of any one of the preceding claims, **characterized in that** the distance from the first surface (11) to the second surface (12) of the carrier material (10) is between 0.5 mm and 10.0 mm, preferably between 1.0 mm and 8.0 mm and more preferably between 1.5 mm and 5.0 mm.

9. The wound dressing (40) of any one of the preceding claims, **characterized in that** the carrier material (10) comprises at least 2, preferably 2 to 50, more preferably 2 to 10 test areas (20).

10. The wound dressing (40) of claim 9, **characterized in that** each of the test areas (20) comprises only one type of test substance (21, 22).

11. The wound dressing (40) of claim 9, **characterized in that** the test areas (20) comprise at least two different types of test substances (21, 22).

12. The wound dressing (40) of any one of the preceding claims, **characterized in that** between 5 % and 90 %, preferably between 10 % and 85 % and more preferably between 20 % and 80 % of the first surface (11) is covered by the at least one test area (20).

13. A method of manufacturing a support layer (1) for a wound dressing (40) according to claim 1, wherein the support layer (1) is adapted for enzymatic detection of an infection in wound exudate, the method comprising the steps of:
- providing a carrier material (10) capable of conducting liquids having a first surface (11) and a second surface (12) opposite the first surface (11),
- applying a barrier composition comprising a hydrophobic substance on the first surface (11), which is capable of forming a diffusion barrier (30) in the carrier material (10), so that a test area (20) confined by said diffusion barrier (30) is formed,
- curing and/or drying the barrier composition,
- applying a test substance (21, 22) to the test area (20) on the first surface (11), such that the test substance (21, 22) is retained within the test area (20) by the diffusion barrier (30).

14. The method of claim 13, **characterized in that** the barrier composition is inkjet printed, plotted, sprayed or coated by slot die, in particular screen printed, onto the carrier material (10).

15. The method of claim 13 or claim 14, **characterized in that** the test substance (21, 22) is applied to the test area (20) by inkjet printing, plotting, spraying or coating by slot die, in particular by drop casting.

## Patentansprüche

1. Wundverband (40) umfassend eine Trägerschicht (1), die zum enzymatischen Nachweisen einer Infektion in einem Wundexsudat ausgelegt ist, wobei die Trägerschicht (1) ein Trägermaterial (10) umfasst, das fähig ist, Flüssigkeiten zu leiten, wobei das Trägermaterial (10) ein absorbierendes Material, ein Papier, ein Hydrogel, einen Vliesstoff, ein Gewebe, einen Schaum oder eine Gaze umfasst oder daraus besteht, wobei das Trägermaterial (10) eine erste Oberfläche (11) und eine zweite Oberfläche (12) gegenüber der ersten Oberfläche (11) aufweist und wobei das Trägermaterial (10) wenigstens einen Testbereich (20) umfasst, auf den eine Testsubstanz (21, 22) aufgebracht ist, wobei das Trägermaterial (10) eine Diffusionsbarriere (30) umfasst, die einen hydrophoben Stoff, ein Polymermaterial und/oder ein Lipid umfasst und seitliche Diffusion der Testsubstanz (21, 22) verhindert, so dass die Testsubstanz (21, 22) durch die Diffusionsbarriere (30) in dem wenigstens einen Testbereich (20) zurückgehalten wird, **dadurch gekennzeichnet, dass** die Diffusionsbarriere (30) rohrförmig von der ersten Oberfläche (11) zu der zweiten Oberfläche (12) des Trägermaterials (10) verläuft.

2. Wundverband (40) nach Anspruch 1, **dadurch gekennzeichnet, dass** die Diffusionsbarriere (30) ein oder mehrere Silicone, insbesondere Polydimethylsiloxan, umfasst.

3. Wundverband (40) nach Anspruch 1 oder Anspruch 2, **dadurch gekennzeichnet, dass** die Diffusionsbarriere (30) auf die erste Oberfläche (11) des Trägermaterials (10) gedruckt, aufgetragen, geprägt, gesprüht oder geschichtet ist, wobei die Diffusionsbarriere (30) vorzugsweise auf die erste Oberfläche (11) des Trägermaterials (10) siebgedruckt ist.

4. Wundverband (40) nach einem der vorstehenden Ansprüche, **dadurch gekennzeichnet, dass** die Diffusionsbarriere (30) den wenigstens einen Testbereich (20) vollständig umgibt.

5. Wundverband (40) nach einem der vorstehenden Ansprüche, **dadurch gekennzeichnet, dass** die Diffusionsbarriere (30) wenigstens 10 %, vorzugsweise wenigstens 50 % und besonders bevorzugt im Wesentlichen vollständig in einer vertikalen Richtung von der ersten Oberfläche (11) zu der zweiten Oberfläche (12) in das Trägermaterial (10) verläuft.

6. Wundverband (40) nach einem der vorstehenden Ansprüche, **dadurch gekennzeichnet, dass** die Dicke (d) der Wand der Diffusionsbarriere (30), gemessen an der ersten Oberfläche (11), zwischen 0,1 mm und 5,0 mm, vorzugsweise zwischen 0,5 mm und 3,0 mm und bevorzugter zwischen 0,6 mm und 2,0 mm beträgt.

7. Wundverband (40) nach einem der vorstehenden Ansprüche, **dadurch gekennzeichnet, dass** die Trägerschicht (1) als Wundauflage gestaltet ist.

8. Wundverband (40) nach einem der vorstehenden Ansprüche, **dadurch gekennzeichnet, dass** der Abstand von der ersten Oberfläche (11) zu der zweiten Oberfläche (12) des Trägermaterials (10) zwischen 0,5 mm und 10,0 mm, vorzugsweise zwischen 1,0 mm und 8,0 mm und bevorzugter zwischen 1,5 mm und 5,0 mm beträgt.

9. Wundverband (40) nach einem der vorstehenden Ansprüche, **dadurch gekennzeichnet, dass** das Trägermaterial (10) wenigstens 2, vorzugsweise 2 bis 50, bevorzugter 2 bis 10 Testbereiche (20) umfasst.

10. Wundverband (40) nach Anspruch 9, **dadurch gekennzeichnet, dass** jeder der Testbereiche (20) nur eine Art von Testsubstanz (21, 22) umfasst.

11. Wundverband (40) nach Anspruch 9, **dadurch gekennzeichnet, dass** die Testbereiche (20) wenigstens zwei verschiedene Typen von Testsubstanzen (21, 22) umfassen.

12. Wundverband (40) nach einem der vorstehenden Ansprüche, **dadurch gekennzeichnet, dass** zwischen 5 % und 90 %, vorzugsweise zwischen 10 % und 85 % und bevorzugter zwischen 20 % und 80 % der ersten Oberfläche (11) von dem wenigstens einen Testbereich (20) bedeckt sind.

13. Verfahren zur Herstellung einer Trägerschicht (1) für einen Wundverband (40) nach Anspruch 1, wobei die Trägerschicht (1) zum enzymatischen Nachweisen einer Infektion in Wundexsudat ausgelegt ist, wobei das Verfahren die Schritte umfasst
- Bereitstellen eines Trägermaterials (10), das fähig ist, Flüssigkeiten zu leiten, mit einer ersten Oberfläche (11) und einer zweiten Oberfläche (12) gegenüber der ersten Oberfläche (11),
- Aufbringen einer Barrierezusammensetzung umfassend eine hydrophobe Substanz, die in der Lage ist, eine Diffusionsbarriere (30) in dem Trägermaterial (10) zu bilden, auf die erste Oberfläche (11), so dass ein durch die Diffusionsbarriere (30) begrenzter Testbereich (20) gebildet wird,
- Härten und/oder Trocknen der Barrierezusammensetzung,
- Aufbringen einer Testsubstanz (21, 22) auf den Testbereich (20) auf der ersten Oberfläche (11), so dass die Testsubstanz (21, 22) von der Diffusionsbarriere (30) innerhalb des Testbereichs (20) zurückgehalten wird.

14. Verfahren nach Anspruch 13, **dadurch gekennzeichnet, dass** die Barrierezusammensetzung auf das Trägermaterial (10) tintenstrahlgedruckt, aufgetragen, gesprüht oder durch eine Schlitzdüse aufgeschichtet, insbesondere siebgedruckt, wird.

15. Verfahren nach Anspruch 13 oder Anspruch 14, **dadurch gekennzeichnet, dass** die Testsubstanz (21, 22) durch Tintenstrahldruck, Auftragen, Sprühen oder Beschichten durch eine Schlitzdüse, insbesondere durch Auftropfen, auf den Testbereich (20) aufgebracht wird.

## Revendications

1. Pansement (40) comprenant une couche support (1) adaptée pour la détection enzymatique d'une infection dans un exsudat de plaie, la couche support (1) comprenant un matériau support (10) capable de conduire les liquides, le matériau support (10) comprenant ou étant constitué par un matériau absorbant, un papier, un hydrogel, un textile non tissé, un textile tissé, une mousse, ou une gaze, le matériau support (10) ayant une première surface (11) et une seconde surface (12) opposée à la première surface (11), et le matériau support (10) comprenant au moins une zone d'essai (20) sur laquelle une substance d'essai (21, 22) est appliquée, le matériau support (10) comprenant une barrière de diffusion (30) qui comprend une substance hydrophobe, un matériau polymère et/ou un lipide et empêche la diffusion latérale de la substance d'essai (21, 22) de telle sorte que la substance d'essai (21, 22) est retenue dans au moins une zone d'essai (20) par la barrière de diffusion (30), **caractérisé en ce que** la barrière de diffusion (30) s'étend de manière tubulaire de la première surface (11) vers la seconde surface (12) du matériau support (10).

2. Pansement (40) selon la revendication 1, **caractérisé en ce que** la barrière de diffusion (30) comprend une ou plusieurs silicones, en particulier du polydiméthylsiloxane.

3. Pansement (40) selon la revendication 1 ou 2, **caractérisé en ce que** la barrière de diffusion (30) est imprimée, tracée, estampée, pulvérisée ou enduite sur la première surface (11) du matériau support (10), de préférence la barrière de diffusion (30) est sérigraphiée sur la première surface (11) du matériau support (10).

4. Pansement (40) selon l'une quelconque des revendications précédentes, **caractérisé en ce que** la barrière de diffusion (30) entoure complètement l'au moins une zone d'essai (20).

5. Pansement (40) selon l'une quelconque des revendications précédentes, **caractérisé en ce que** la barrière de diffusion (30) s'étend d'au moins 10 %, de préférence d'au moins 50 %, et de manière particulièrement préférée sensiblement complètement dans le matériau support (10) depuis la première surface (11) jusqu'à la seconde surface (12) dans une direction verticale.

6. Pansement (40) selon l'une quelconque des revendications précédentes, **caractérisé en ce que** l'épaisseur (d) de la paroi de la barrière de diffusion (30) mesurée au niveau de la première surface (11) est comprise entre 0,1 mm et 5,0 mm, de préférence entre 0,5 mm et 3,0 mm et plus préférablement entre 0,6 mm et 2,0 mm.

7. Pansement (40) selon l'une quelconque des revendications précédentes, **caractérisé en ce que** la couche support (1) est réalisée sous la forme d'un coussinet de plaie.

8. Pansement (40) selon l'une quelconque des revendications précédentes, **caractérisé en ce que** la distance entre la première surface (11) et la seconde surface (12) du matériau support (10) est comprise entre 0,5 mm et 10,0 mm, de préférence entre 1,0 mm et 8,0 mm et plus préférablement entre 1,5 mm et 5,0 mm.

9. Pansement (40) selon l'une quelconque des revendications précédentes, **caractérisé en ce que** le matériau support (10) comprend au moins 2, de préférence 2 à 50, plus préférablement 2 à 10 zones d'essai (20).

10. Pansement (40) selon la revendication 9, **caractérisé en ce que** chacune des zones d'essai (20) comprend un seul type de substance d'essai (21, 22).

11. Pansement (40) selon la revendication 9, **caractérisé en ce que** les zones d'essai (20) comprennent au moins deux types différents de substances d'essai (21, 22).

12. Pansement (40) selon l'une quelconque des revendications précédentes, **caractérisé en ce qu'**entre 5 % et 90 %, de préférence entre 10 % et 85 % et plus préférablement entre 20 % et 80 % de la première surface (11) est recouverte par l'au moins une zone d'essai (20).

13. Procédé de fabrication d'une couche support (1) pour un pansement (40) selon la revendication 1, la couche support (1) étant adaptée pour la détection enzymatique d'une infection dans l'exsudat de plaie, le procédé comprenant les étapes de
- fourniture d'un matériau support (10) capable de conduire les liquides ayant une première surface (11) et une seconde surface (12) opposée à la première surface (11),
- application d'une composition barrière comprenant sur la première surface (11) une substance hydrophobe, apte à former une barrière de diffusion (30) dans le matériau support (10), de sorte qu'est formée une zone d'essai (20) confinée par ladite barrière de diffusion (30),
- durcissement et/ou séchage de la composition barrière,
- application d'une substance d'essai (21, 22) sur la zone d'essai (20) sur la première surface (11), de telle sorte que la substance d'essai (21, 22) est retenue dans la zone d'essai (20) par la barrière de diffusion (30).

14. Procédé selon la revendication 13, **caractérisé en ce que** la composition barrière est imprimée par jet d'encre, tracée, pulvérisée ou enduite par une filière à fente, en particulier sérigraphiée, sur le matériau support (10).

15. Procédé selon la revendication 13 ou 14, **caractérisé en ce que** la substance d'essai (21, 22) est appliquée sur la zone d'essai (20) par impression à jet d'encre, traçage, pulvérisation ou enduction par filière à fente, en particulier par coulée goutte à goutte.
